# EUROPEAN PATENT APPLICATION

(11) **EP 4 470 590 A1**
(43) Date of publication of application: **04.12.2024**
(21) Application number: 24175265.8
(22) Date of filing: 10.05.2024
(51) Int. Cl.: A61M 16/04, A61M 16/08, A61M 16/16, A61M 16/10, A61M 16/00

(54) **ADAPTER AND SYSTEM FOR VENTILATION SUPPORT**

(30) Priority: 30.05.2023 US 202318325870
(71) Applicant: GE Precision Healthcare LLC, Waukesha, WI 53188 (US)
(72) Inventor: HIETALA, Mika Harri Juhani, 00510 Uusimaa (FI)
(74) Representative: Fennell, Gareth Charles

(57) **Abstract**

An adapter is provided for a ventilation system. The adapter (300) comprises a junction (302) with an integrated region (304) for directly releasably receiving a gas concentration sensor, a first port (306) fluidly coupled to the junction configured to interface with a ventilator piece, a second port (308) configured to interface with a suction catheter system, and a third port (310) configured to interface with an intubation tube.

## Description

### FIELD

Embodiments of the subject matter disclosed herein relate to assisted ventilation of a subject.

### BACKGROUND

During an event where ventilation support is demanded for a subject, such as a patient, undergoing a surgery or a procedure which requires anesthetization, a ventilation system may be used to provide requisite pulmonary gas exchanges to sustain life. The ventilation system may have a relatively complex configuration for delivering oxygen to and removing carbon dioxide from the subject's lungs and may rely on microprocessor-based control of sensors, valves, flow rate controllers, closed or open suction catheters, and various other components. The sensors, intubation tubing, and suction catheters may be connected to the ventilation system by one or more adapters. The subject may thereby be mechanically ventilated and flow of gases to and from the subject may be monitored and controlled by the ventilation system.

### BRIEF DESCRIPTION

In one example, an adapter for a ventilation system comprises a junction with an integrated region for directly releasably receiving a gas concentration sensor, a first port fluidly coupled to the junction configured to interface with a ventilator piece, a second port configured to interface with a suction catheter system, and a third port configured to interface with an intubation tube.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present invention will be better understood from reading the following description of non-limiting embodiments, with reference to the attached drawings, wherein below:
FIG. 1 shows an example of a ventilation system for providing respiratory support to a subject.
FIG. 2 shows a schematic diagram of a ventilation system for providing respiratory support to a subject.
FIG. 3 shows a gas sensor adapter with suction port.
FIG. 4 shows a gas concentration sensor mounted to the gas sensor adapter with suction port.
FIG. 5 shows a ventilator y-piece and the gas concentration sensor coupled to the gas sensor adapter with suction port.
FIG. 6 shows the ventilator y-piece, the gas concentration sensor, and a suction catheter system coupled to the gas sensor adapter with suction port.
FIG. 7 shows a second example of a gas sensor adapter with suction port.
FIG. 8 shows a use example including a gas sensor adapter with suction port.
FIG. 9 shows a detail view of the second example of a gas sensor adapter with suction port.
FIG. 10 shows another detail view of the second example of a gas sensor adapter with suction port.

### DETAILED DESCRIPTION

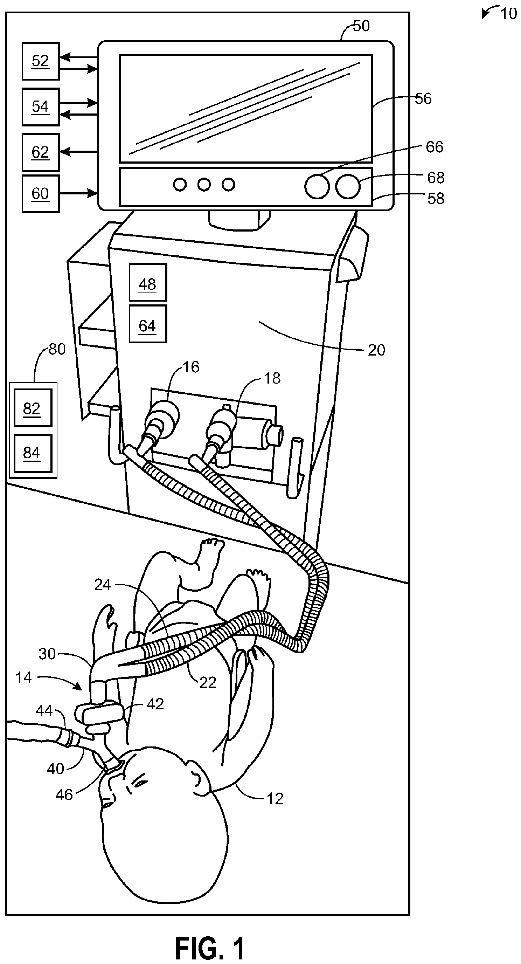

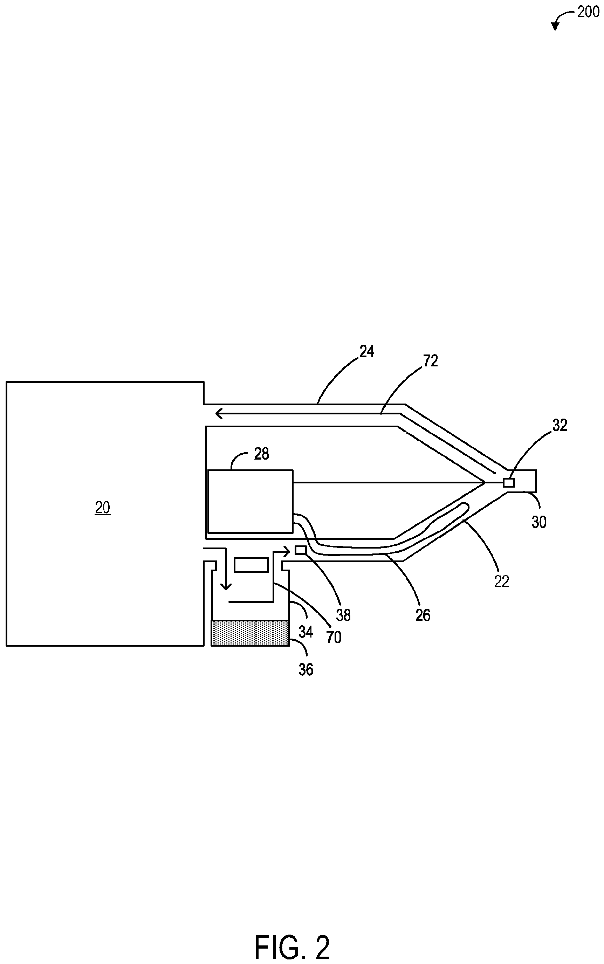

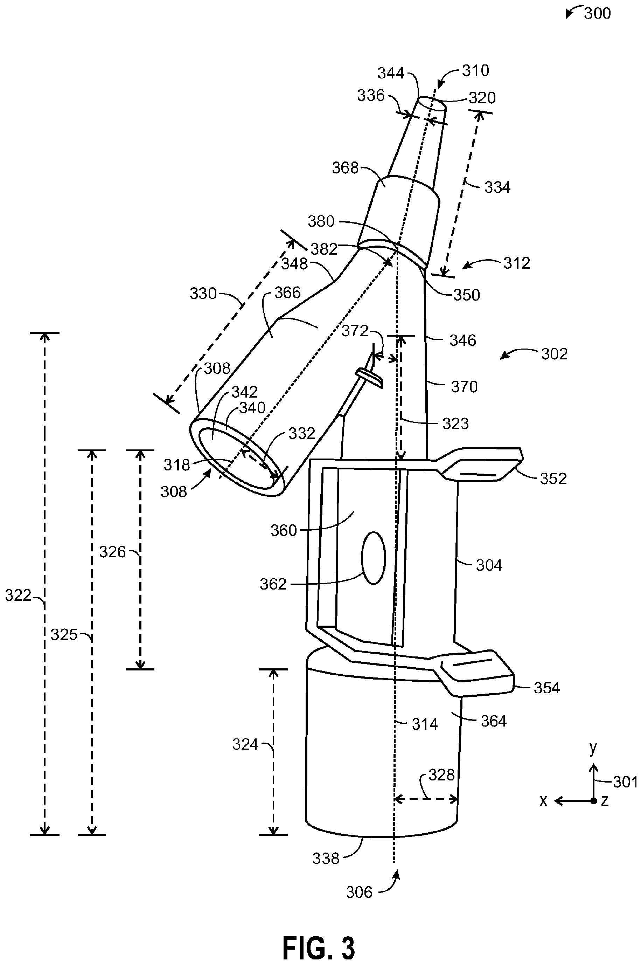

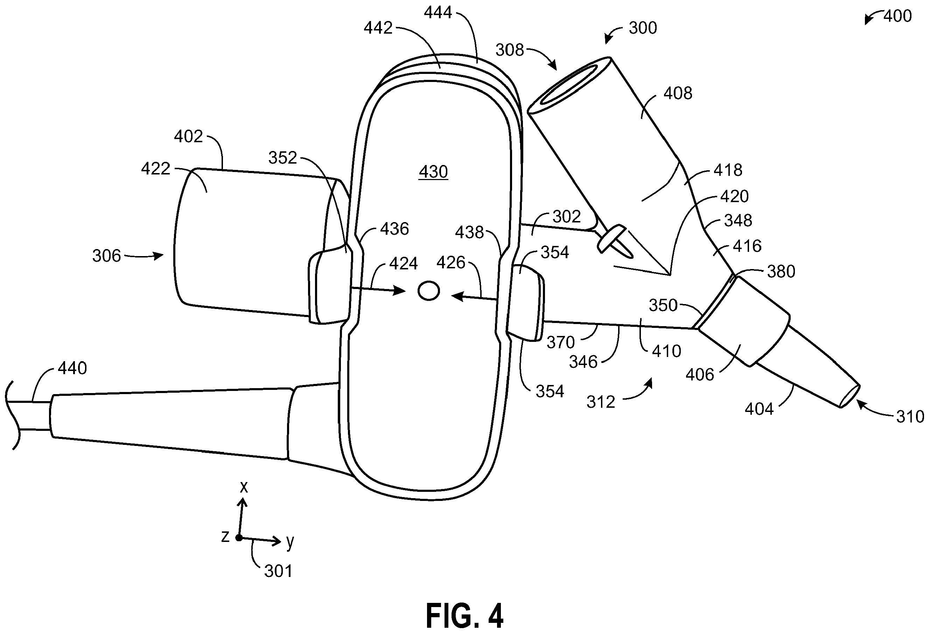

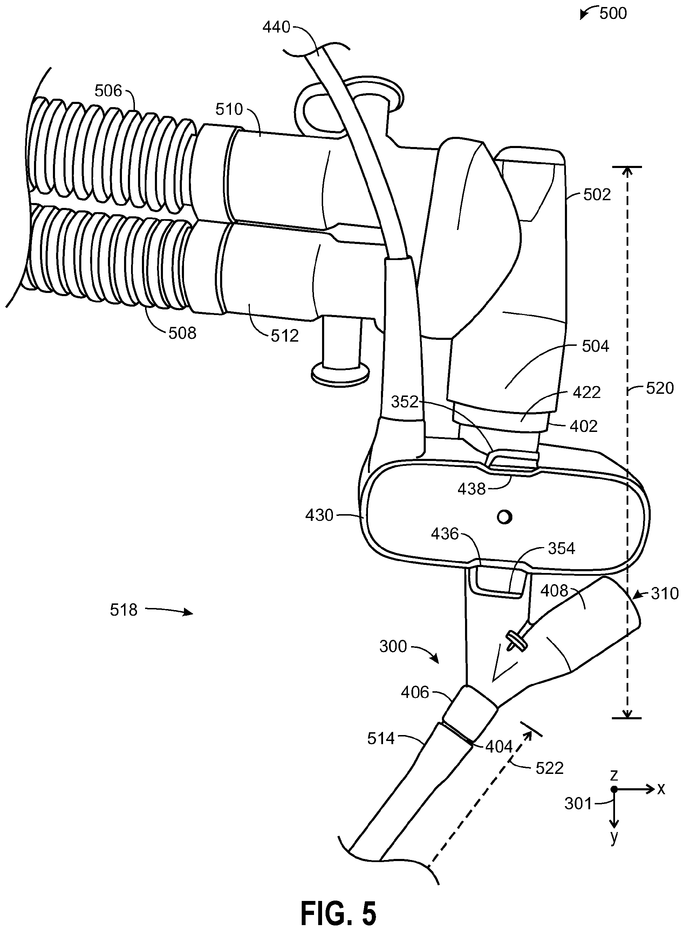

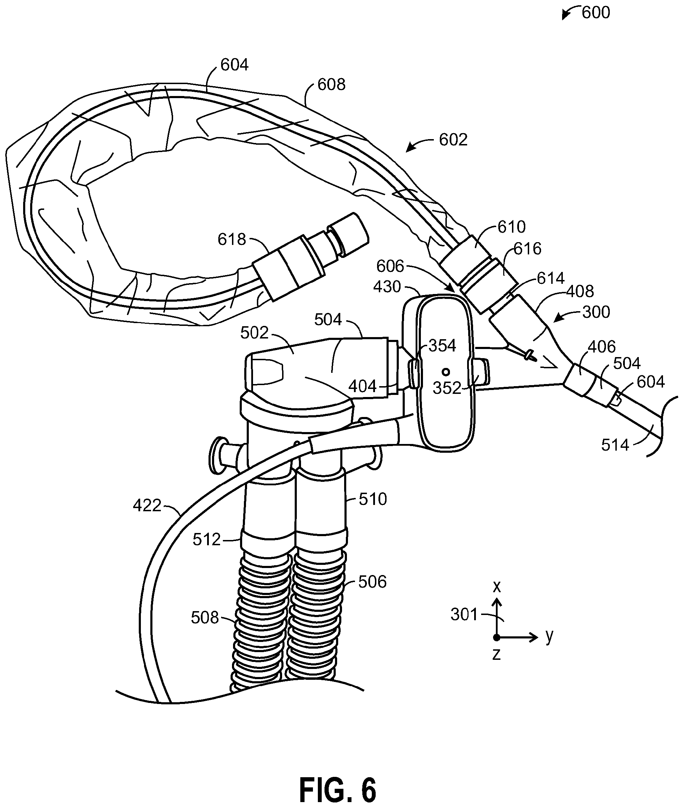

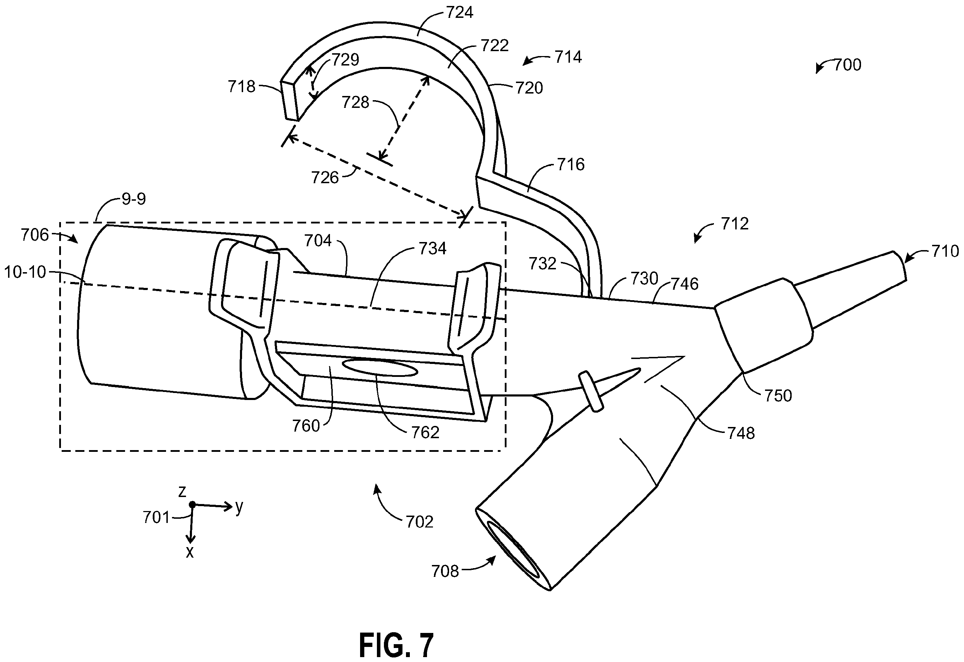

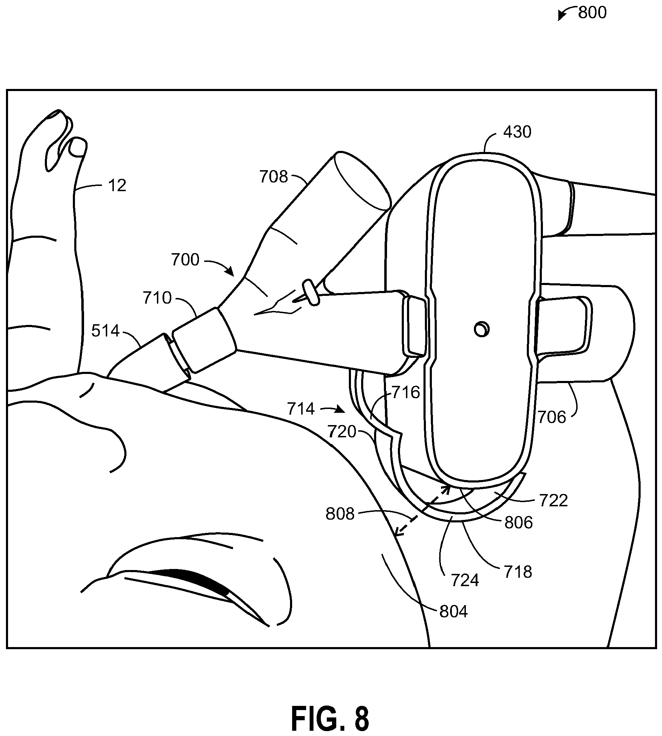

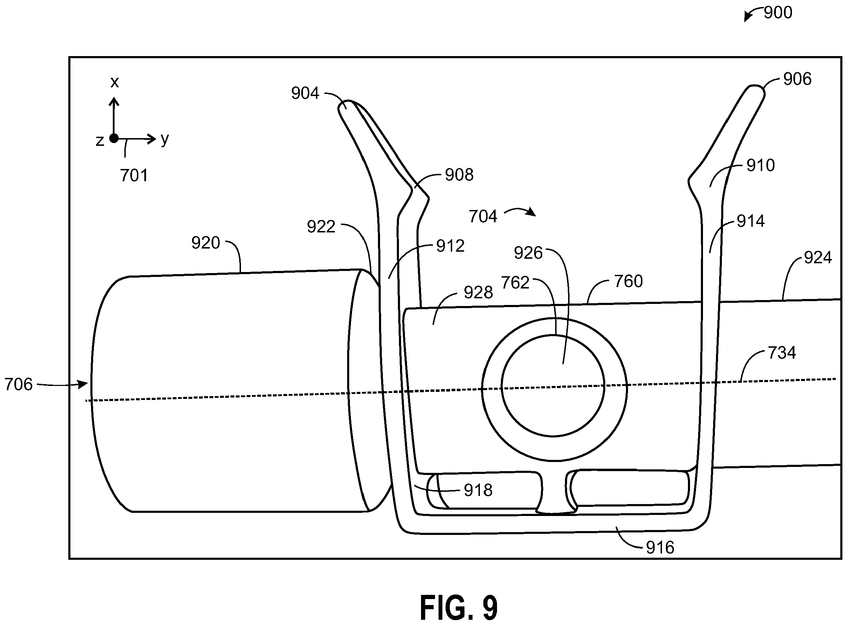

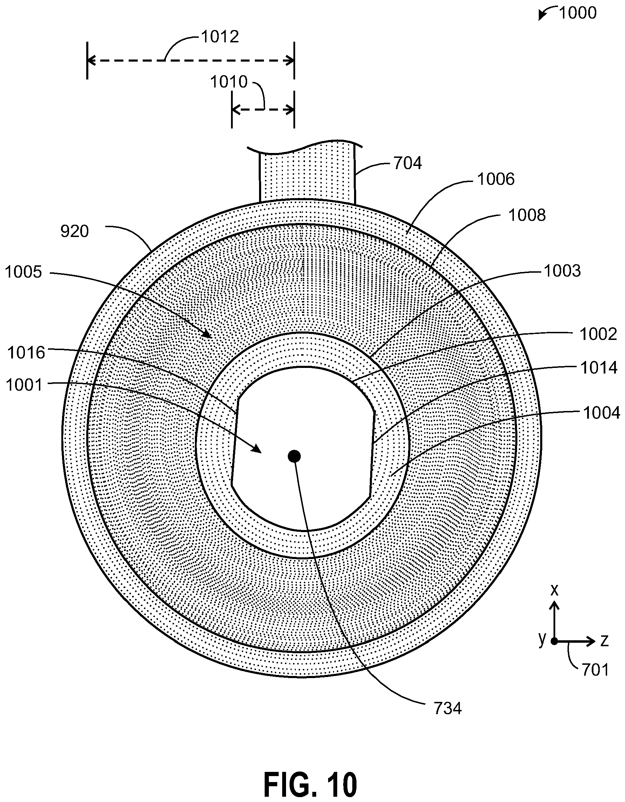

Aspects of the present disclosure will now be described, by way of example, with reference to FIGS. 1-10, which relate to an adapter for a mechanical ventilation system. An example of a ventilation system enabling mechanical ventilation support is shown in FIG. 1. In one example, the ventilation system is a neonate ventilation system. FIG. 2 shows a schematic representation of a ventilation system, which may be the same or similar to the ventilation system shown in FIG. 1. An adapter for a ventilation system is shown in FIG. 3. The adapter is a gas sensor adapter with suction port. The adapter is shown in FIG. 4 with a gas sensor mounted thereto. The adapter is shown in FIG. 5 with the gas sensor mounted thereto and coupled to a ventilator y-piece. The adapter is shown in FIG. 6 coupled to the gas sensor, the ventilator y-piece, and a suction catheter system. Gas concentration sensors produce heat that, in some examples, may damage sensitive patient skin, particularly neonate patients. A second example of an adapter including a heat guard designed to protect the patient from gas sensor heat is shown in FIG. 7. The second example of the adapter in a use example with a patient is shown in FIG. 8. FIG. 9 and FIG. 10 are detail views of the second example of the adapter.

Medical gas (e.g., purified fresh air, O₂ rich) may be delivered to a patient using a mechanical ventilation system. Adapters may be used with the ventilation system for connecting intubation (or endotracheal) tubing, sensors, and suction catheter systems. One example of a sensor that may be included in ventilation circuitry is a gas sensor adapter for monitoring patient end tidal CO₂ (ETCO₂). End tidal CO₂ measurement is a method for monitoring respiratory status during ventilation. Other methods include pulse oximetry, arterial CO₂ monitoring, and transcutaneous CO₂ monitoring. Suction catheter systems may be included in ventilation systems for management of bronchial secretions in intubated patients. Suction catheter systems include open systems and closed systems. Open systems may include, for example, systems where a patient is disconnected from the ventilator during secretion removal and systems where suction is performed through a selectively opened accessory port by inserting a suction tube through the port. After suctioning, the suction tube is removed and the port sealed making the system airtight again. Closed suction systems include systems where the patient remains connected during secretion removal.

A gas sensor adapter for measuring ETCO₂ is a separate component from a suction catheter adapter, both of which may be joined to the intubation tube. The gas sensor adapter and the suction catheter adapter may be joined to the intubation tube in series. The positioning of the gas sensor adapter and the suction catheter adapter presents challenges for the ventilation circuitry. For example, arranging the gas sensor close to the end of the intubation tube is desirable for accurate gas measurement. However, if the gas sensor is closer to the intubation tube than the suction catheter, in some examples, the suction may be performed through the mainstream and suction tube may interfere with the gas measurement. Particularly, the suction system may introduce contaminants to the sensor compromising the accuracy of the measurement. Arranging the gas sensor adapter and the suction catheter adapter on separate ports may reduce contamination; however, such an arrangement may increase mechanical dead space in the ventilation circuitry.

Mechanical dead space, which may be the volume of ventilated air that flows in both directions as the subject breathes in and out but not participating in gas exchange, may increase a proportion of CO₂ inhaled by the subject and may interfere with accurate readings of end tidal CO₂. Mechanical dead space mainly comprises the connection between the mainstream adapter and the patient (e.g. the intubation tube, a humidifier/heat exchanger, adapters for measuring gas contents, flow, pressure etc.) Dead space can be relatively large, depending on the design of the ventilation system.

Mechanical dead space is particularly challenging for neonatal patients, who have relatively small tidal volumes. Tidal volume is an amount of air that moves in and out of lungs during a respiratory cycle, which for neonatal patients may be in the range of 6-8 mL compared to a healthy adult, which may average between 400 mL and 500 mL. As the use of a gas sensor adapter for measuring ETCO₂ may increase mechanical dead space, more commonly transcutaneous CO₂ and arterial CO₂ monitoring are employed in the ventilation assessment of neonate patients. However, transcutaneous CO₂ sensors are hot and may cause burns for neonate patient skin if used for long periods, and arterial CO₂ monitoring may be invasive, particularly for neonates with relatively small blood volumes.

The herein disclosed adapter for a ventilation system provides in a single adapter connections for a gas concentration sensor, a suction catheter system, and intubation tubing, with reduced mechanical dead space. The position of the gas concentration sensor achieves accurate reading and reduces contamination from the suction catheter. Reduction of mechanical dead space increases accurate delivery of medical gas to the patient and increases the accuracy and sensitivity of the gas sensor measurement. By integrating the gas measurement and suction catheter adapters into a single adapter, a lighter and smaller packaging is achieved. Additionally, by providing a connections for the gas concentration sensor and closed suction catheter in a single adapter, more invasive methods of CO₂ measurement in the subject may be avoided.

Before further discussion of the adapter for a mechanical ventilator, a general description of a medical system configured to provide ventilation support is provided. It should be understood that the various examples are not limited to the arrangements and instrumentality shown in the drawings.

An example of a ventilation system 10 that may to provide ventilation support to a subject is shown in FIGS. 1-2. The ventilation system 10 may be used to provide pulmonary gas exchanges to sustain life during a procedure, such as a surgery, which requires anesthetization, or for an extended or chronic condition. As one example, the ventilation system 10 may be a neonate ventilation system.

FIG. 1 shows the ventilation system 10 including a source of medical gas 64, a ventilator 20, a gas concentration sensor 42, a suction catheter system 80, an intubation tube 46, and a gas sensor adapter with suction port or adapter 40. The adapter 40 comprises a junction with an integrated region for directly releasably receiving a gas sensor, such as the gas concentration sensor 42. The adapter 40 is a y-shaped manifold having ports configured to interface with an intubation tube, a ventilator, and a closed suction catheter system. In the ventilation system 10, the adapter 40 is coupled to the ventilator 20, the gas concentration sensor 42, the suction catheter system 80, and the intubation tube 46.

The ventilator 20 includes a breathing circuit 14 comprising an inspiratory branch 22, an expiratory branch 24, and the intubation tube 46. The ventilator 20 and the breathing circuit 14 may cooperate to provide breathing gases to a patient 12. In one example, the patient 12 is a neonatal patient. The inspiratory branch 22 is coupled to the ventilator 20 via a first coupling 16. The expiratory branch is coupled to the ventilator 20 via a second coupling 18. In one example, one end of each of the inspiratory branch 22 and expiratory branch 24 is connected to the ventilator 20, while the other ends thereof are connected to a ventilator piece, which can then connect to the patient 12 through the adapter 40. In one non-limiting example, the ventilator piece is a ventilator y-piece 30 of the breathing circuit 14. In other examples, the ventilator piece is any other accessory between the adapter 40 and a ventilator Y-piece of a breathing circuit, such as a flow sensor. The intubation tube 46 is fluidly coupled to the adapter 40 and the intubation tube 46 inserted into the end of airways of the patient 12. The breathing circuit 14 may be decoupled from the adapter 40 for replacing the adapter in the event the adapter is degraded, which is described in more detail following FIG. 10.

The gas concentration sensor 42 is releasably mounted to the adapter 40 for sampling gas concentration in the breathing circuit 14. In one example, the gas concentration sensor 42 may use an infrared absorption (IR) technique. In other examples, the gas concentration sensor 42 may be a mainstream or multi-function sensor for measuring to the patient fractional concentration of inspired CO₂ (FiCO₂), ETCO², real-time CO₂ concentration (e.g., capnogram), and respiration rate. Additionally, a multi-function sensor may measure nitrous oxide (N₂O) and anesthetic agents, and/or flow. Similarly, the gas concentration sensor 42 may be decoupled from the adapter 40 and replaced with a second adapter in the event the adapter is degraded.

The suction catheter system 80 is coupled to the adapter 40. The suction catheter system 80 may be a closed suction catheter system that remains coupled to the patient 12 during ventilation. The suction catheter system 80 may include a pump 82 that in communication with a suction catheter 44 and a suction catheter controller 84 may be operated for management of bronchial secretion and biofilm accumulation in the patient 12 and the intubation tube 46. Similarly, the suction catheter system 80 may be decoupled from the adapter 40 for replacing the adapter in the event the adapter is degraded.

The ventilator 20 may also include a control system 50. The control system 50 may control various pneumatic elements of the ventilator 20 to provide breathing gases to the lungs of the patient 12 through the inspiratory branch 22 of the breathing circuit 14. The breathing gases may be discharged from the lungs of the patient 12 and into the expiratory branch 24 of the breathing circuit 14. This process can be iteratively enabled by the control system 50 of the ventilator 20, which can establish various control parameters, such as the number of breaths per minute to administer to the patient 12, tidal volumes (V_{T}), maximum pressures, etc., that can characterize the mechanical ventilation that the ventilator 20 supplies to the patient 12. As such, the ventilator 20 may be microprocessor-based and operable in conjunction with a suitable memory to control pulmonary gas exchanges in the breathing circuit 14 connected to, and between, the patient 12 and the ventilator 20. In one example, the control system 50 includes a processor 52, a memory 54, a monitor 56, and a user interface 58.

The control system 50 may include various electronic components, e.g., hardware, for receiving and transmitting signals, and processing thereof. For example, the processor 52 may be configured to receive signals from a plurality of sensors 60, which may include pressure sensors, flow sensors, sensors monitoring statuses of valves and switches, etc., and send control signals to a plurality of actuators 62 of the medical system, such as pressure regulators, the valves and switches, etc., in response to the sensor signals. The memory 54 may be an electronic storage medium (including non-transitory memory) for storing executable programs and parameter setting values.

The ventilation system 10 may provide the breathing gases directly to the lungs of the patient 12, as may be used in a chronic and/or critical care application, or the ventilation system 10 may provide a driving gas to compress a bellows 48 (as shown in FIG. 1) containing the breathing gases. In turn, the bellows 48 may supply the breathing gases to the lungs of the patient 12, such as in an anesthesia application. In either case, the breathing gases may iteratively pass from the inspiratory branch 22, to the ventilator y-piece 30 and to the patient 12, and then back to the ventilator 20 via the ventilator y-piece 30 and expiratory branch 24.

The ventilation system 10 may receive inputs from the sensors 60 associated with the ventilator 20 at the control system 50 for subsequent processing thereof. The processed inputs may be displayed on the monitor 56. Representative data received from the sensors 60 may include, for example, inspiratory time (T_{I}), expiratory time (T_{E}), natural exhalation time (T_{EXH}), respiratory rates (f), I:E ratios, positive end expiratory pressure (PEEP), fractional inspired oxygen (F_{I}O₂), fractional expired oxygen (F_{E}O₂), breathing gas flow (F), tidal volumes (V_{T}), temperatures (T), airway pressures (P_{aw}), arterial blood oxygen saturation levels (SₐO₂), blood pressure information (BP), pulse rates (PR), pulse oximetry levels (SₚO₂), concentration of inspired inhalation anesthetic agent (Cr agent), concentration of expired inhalation anesthetic agent (C_{E} agent), arterial blood oxygen partial pressure (PₐO₂), arterial carbon dioxide partial pressure (PₐCO₂), and the like. For example, the control system 50 may receive from the gas concentration sensor 42 signals related to ETCO₂ measurement.

The control system 50 of the ventilator 20 may also control displaying numerical and/or graphical information from the breathing circuit 14 on the monitor 56 of the ventilation system 10, as well as other patient 12 and/or ventilation system 10 parameters from the sensors 60. In other examples, various components can also be integrated and/or separated, as needed and/or desired. The control system 50 may also be configured to display signals for the monitor 56 and/or the like, control alarms 66, and/or the user interface 58, which may include a graphical user interface (GUI) displayed at the monitor 56, and one or more input devices 68, etc., all as demanded and/or desired and interconnected suitably.

Turning to FIG. 2, a schematic diagram 200 of the ventilation system 10 is shown. The ventilator 20 is fluidly connected to the ventilator y-piece 30 via the inspiratory branch 22 and the expiratory branch 24. Medical gas 70 (e.g., purified fresh air, O2 rich, breathing gas) is delivered from ventilator 20 to the patient through the inspiratory branch 22. Expirated medical gas 72 (e.g., respired breath) is delivered to the ventilator 20 from the patient through the expiratory branch 24. The ventilation system 10 includes a heated wire controller 28 operating a heated wire circuit 26 in electronic communication with an airway temperature sensor 32. The heated wire controller 28 may control the heated wire circuit 26 to warm the medical gas 70 passing through the inspiratory branch 22 in response to signals received from the airway temperature sensor 32. The ventilation system 10 includes a humidifier controller 34 operating a humidifier 36 in electronic communication with a humidity sensor 38. The humidifier controller 34 may control the humidifier 36 to moisturize the medical gas 70 passing through the inspiratory branch 22 in response to signals received from the humidity sensor 38. In one example, the control system 50 may be in electronic communication with the humidifier controller 34, the heated wire controller 28, the airway temperature sensor 32, and the humidity sensor 38.

FIG. 3 shows a gas sensor adapter with suction port, hereinafter an adapter 300, for a ventilation system. The adapter 300 integrates into a single adapter connections for a gas concentration sensor, a closed suction catheter system, and intubation tubing, and, in doing so, reduces the dimension, weight, and mechanical dead space that challenge existing gas sensor adapter and suction catheter adapter configurations. The adapter 300 may be the same or similar to the adapter 40 described with reference to FIG. 1, which may be included in an ventilation system (e.g., the ventilation system 10 shown in FIGS. 1-2). An axis system 301 is given in FIG. 3 and the figures following indicating an x-axis, a y-axis, and a z-axis.

The adapter 300 comprises a junction 302 with an integrated region 304 for directly releasably receiving a gas concentration sensor (e.g., gas concentration sensor 42). The adapter 300 comprises a first port 306 fluidly coupled to the junction 302 configured to interface with a ventilator piece, a second port 308 configured to interface with suction catheter system, and a third port 310 configured to interface with an intubation tube (e.g., the gas concentration sensor, the ventilator y-piece 30, the suction catheter system 80, and the intubation tube 46 in FIG. 1). In one example, the adapter 300 is a one-piece, monolithic member.

The adapter 300 may comprise a manifold 312, wherein the junction 302, the second port 308, and the third port 310 branch off from the manifold 312 to form separate channels. In one example, the junction 302, the second port 308, and the third port 310 branch off from the manifold 312 to form a y-shape. For example, the junction 302 branches off from the manifold 312 at a junction branch 346. The second port 308 branches off from the manifold 312 at a second port branch 348. The third port 310 branches off from the manifold 312 at a third port branch 350. In one example, the junction 302 and the second port 308 branch off from the manifold 312 substantially in parallel. In other examples, the branching may form a t-shape. The manifold 312 includes a junction length 370 interposed between the integrated region 304 and the third port 310.

In one example, the integrated region 304 includes a sensor mount surface 360 interposed between a first clip 352 and a second clip 354. When included in a ventilation system, the first clip 352 and the second clip 354 releasably mount the gas concentration sensor to the sensor mount surface 360 and position the sensor over a sensor window 362. For example, the first clip 352 and the second clip 354 may conform to and compressingly make face sharing contact with a surface of the gas concentration sensor. An example of the integrated region including the sensor mount surface is shown in more detail with reference to FIG. 7 and FIG. 9.

The first port 306 may be defined by an opening on a first port surface 338 and a first port interior surface (not shown) that extends from the first port surface 338 to the base of the integrated region 304 (e.g., below the second clip 354). A first port exterior surface 364 extends between the first port surface 338 and the sensor mount surface 360, e.g., to the second clip 354. The second port 308 may be defined by an opening on a second port surface 340 and a second port interior surface 342 that extends from the opening on the second port surface 340 to the second port branch 348. A second port exterior surface 366 extends between the second port surface 340 and the second port branch 348. The third port 310 may be defined by an opening on a third port surface 344 and a third port interior surface (not shown) that extends from the opening on the third port surface 344 to the third port branch 350. A third port exterior surface 368 extends between the third port surface 344 and the third port branch 350.

The junction 302 and the first port 306 have a first port centerline axis 314 that extends through the first port 306, the integrated region 304, and the junction length 370. Extending radially from the first port centerline axis 314 is an inner channel enclosed by an inner pipe wall (see FIG. 10). The second port 308 has a second port centerline axis 318. The third port 310 has a third port centerline axis 320. The first port centerline axis 314 intersects with the second port centerline axis 318 and the third port centerline axis 320. Airflow within the branches converge at an intersection 380 of the first port centerline axis 314, the second port centerline axis 318, and the third port centerline axis 320. Airflow though the branches of the manifold 312 may be parallel to the respective centerline axes. In some examples, an angle 382 formed at the intersection 380 of the first port centerline axis 314 and the second port centerline axis 318 may range from approximately 0 to 90 degrees.

By integrating the sensor mount surface 360 into the junction 302, the adapter 300 reduces a length and a volume of mechanical dead space in the airway. In one example, a first axial length 323 of the junction length 370 is less than a second axial length 334 of the third port 310. The first axial length 323 of the junction length 370 extends from the first clip 352 of the integrated region 304 to the junction branch 346. The second axial length 334 of the third port 310 extends from the third port surface 344 to the third port branch 350. This is a substantial reduction in total length from the third port surface 344 to the sensor mount surface 360 over other related configurations.

Other dimensions of the adapter 300 include a junction branch axial length 322 that extends from the junction branch 346 to the first port surface 338. The integrated region 304 and the first port 306 have a junction length 325 that extends from the first clip 352 to the first port surface 338. The first port 306 has a first port axial length 324 that extends from the first port surface 338 to the second clip 354. The integrated region 304, including the sensor mount surface 360, has a sensor mount axial length 326 that extends from the first clip 352 to the second clip 354. The second port 308 has a second branch axial length 330 that extends from the second port surface 340 to the second port branch 348.

In one example, a cross sectional area of the manifold 312 decreases from the integrated region 304 of the junction 302 to the third port 310. For example, the integrated region 304 has a junction radius 328 through the sensor mount axial length 326. The inner channel of the junction 302 has a smaller radius, which is described with reference to FIG. 10. The junction 302 has a junction branch radius 372 at the junction branch 346. The junction branch radius 372 is less than the junction radius 328. The third port 310 has a third port radius 336. The third port radius 336 is less than the junction branch radius 372. Moreover, lengths of interior passage within the manifold 312 between the aforementioned radii do not increase, but rather decrease over the first axial length 323 and the second axial length 334. Consequently, the cross sectional area decreases from the integrated region 304 (e.g., the sensor mount) through the junction length 370 to the third port 310. A second port radius 332 is also shown, which is more than the third port radius 336. Lengths of interior passage within the manifold 312 similarly decrease over the second branch axial length 330 and the second axial length 334. By reducing mechanical dead space between the integrated region 304 and the third port 310, the adapter 300 substantially reduces total mechanical dead space volume within the manifold 312 over other related configurations.

FIG. 4 shows an assembly 400 including the adapter 300. The adapter 300 may be the same or similar to the adapter 40 described with reference to FIG. 1, which may be included in a ventilation system (e.g., the ventilation system 10 shown in FIGS. 1-2). Components of the adapter 300 introduced with reference to FIG. 3 are numbered the same and will not be reintroduced. Further, some components of the adapter 300 may not be shown, although it may be understood that they may also be included in the adapter 300.

The first port 306 includes an accessory mount 402 and an accessory mount surface 422. In one example, when coupled to the adapter 300, a ventilator y-piece (e.g., ventilator y-piece 30 in FIG. 1) and the accessory mount surface 422 make face sharing contact. For example, the ventilator y-piece may slide over the accessory mount 402, sealingly coupling an airway of the ventilator y-piece and the first port 306. In other examples, an accessory, such as a flow sensor, may be mounted to the accessory mount 402 and the ventilator y-piece coupled to the accessory. The third port 310 comprises an intubation tube mount 404 and a sleeve 406. The sleeve 406 forms a radial thickening around the intubation tube mount 404. When coupled to the adapter 300, an intubation tube (e.g., intubation tube 46 in FIG. 1) and the intubation tube mount 404 make face sharing contact. For example, the intubation tube may slide over the intubation tube mount 404, abutting the sleeve 406, thereby sealingly coupling an airway of the intubation tube and the third port 310. The second port 308 comprises a catheter receiving collar 408. When coupled to the adapter 300, a suction catheter (e.g., suction catheter 44 in FIG. 1) and the catheter receiving collar 408 make face sharing contact. For example, the catheter receiving collar 408 may slide over the suction catheter, sealingly coupling an airway of the suction catheter and the second port 308.

The manifold 312 includes a plurality of interior passages or chambers wherein airflows branch and converge. For example, airflows through a first passage 410 from the third port branch 350 to the junction branch 346 combine (e.g., mix, converge) in the manifold 312 with airflows in a parallel, second passage 416 from the third port branch 350 to the second port branch 348 at the intersection 380. At fluid divergence point 420, airflows through the manifold 312 to the first port 306 diverge substantially in parallel with airflows through the manifold 312 to the second port 308. For example, the fluid divergence point 420 is approximately parallel with the junction branch 346 and the second port branch 348. Airflows through the junction 302 are not combined with airflows through a third passage 418 after the fluid divergence point 420. There is practically no flow to the second port 308 except when suctioning.

The assembly 400 includes a sensor 430 releasably mounted to the adapter 300. The sensor 430 includes a first notch 436 and an opposing, second notch 438. The sensor 430 includes a sensor cable 440. In one example, the sensor cable 440 may transmit electronic signals to a ventilator control system (e.g., control system 50 in FIG. 1).

The sensor 430 mounts over the sensor mount surface 360 of the integrated region 304 (see FIG. 3). The first clip 352 and the second clip 354 fix the sensor 430 to the sensor mount surface 360. For example, the first clip 352 flexibly presses against the first notch 436 in a first direction indicated by arrow 424. The second clip 354 flexibly presses against the second notch 438 in an opposing, second direction indicated by arrow 426. To release the sensor 430, a user presses in the opposite direction indicated by the arrows. In this way, the first clip 352 and the second clip 354 secure the sensor to the adapter 300 and enable quick release of the sensor 430 from the adapter 300 when a replacement adapter is demanded.

In one example, the sensor 430 may be a gas concentration sensor for measuring ETCO₂. For example, the sensor 430 may be an infrared sensor using an infrared absorption (IR) technique. The IR technique relies on the principle that CO₂ molecules absorb infrared light energy at specific wavelengths, with the amount of energy absorbed being directly proportional to the CO₂ concentration. When an IR light beam is passed through a gas sample containing CO₂, an electronic signal from a photodetector can be obtained. In one example, the sensor may 430 sealingly clamp around the sensor window 362 (in FIG. 3). The sensor 430 may include an emitter 442 that generates the IR light beam and a detector 444 that measures the amount of energy absorbed by the gas sample. The IR light beam is passed through the sensor window 362. The light that reaches the detector 444 is used to measure CO₂ concentration. In another example, the sensor 430 may be a CO₂ mainstream or multi-function sensor for measuring to the patient fractional concentration of inspired CO2 (FiCO2), ETCO2, and respiration rate.

Without an integrated adapter, as in related configurations, there are tradeoffs related to the relative placement of a gas sensor, a suction catheter, and the manifold passages leading thereto. Ideally, a gas concentration sensor is placed as close as possible to the end of intubation tube to have a good gas sample to measure. Suction catheters can introduce moisture and other contaminants into a mainstream adapter. If the gas adapter is arranged closest to the intubation tube, then suction is performed through a mainstream adapter. In such an arrangement, the moisture and other contaminants may make the sensor windows of the gas adapter permanently dirty, demanding frequent replacement of the gas adapter, such as following each operation of the suction catheter. If the suction adapter is arranged closest to the end of intubation tube, then the gas adapter position is compromised, and consequently, the gas sampling may produce misleading or inaccurate EtCO2 readings. When coupled in a ventilation system, the parallel branching at the fluid divergence point 420 of the first passage 410, which leads to the sensor 430, and the second passage 416, which leads to a suction catheter system (e.g., see FIG. 6), provides a good position for both suction and gas measurement.

FIG. 5 shows an assembly 500 including the adapter 300. The adapter 300 may be the same or similar to the adapter 40 described with reference to FIG. 1, which may be included in an ventilation system (e.g., the ventilation system 10 shown in FIGS. 1-2). Components of the adapter 300 introduced with reference to FIGS. 3-4 are numbered the same and will not be reintroduced. Further, some components of the adapter 300 may not be shown, although it may be understood that they may also be included in adapter 300.

A ventilator y-piece 502 is coupled the adapter 300. The ventilator y-piece 502 includes a y-piece sleeve 504. The y-piece sleeve 504 is positioned over the accessory mount 402 such that an inner face (not shown) of the y-piece sleeve 504 makes face sharing contact with the accessory mount surface 422, thereby fluidly coupling the adapter 300 to the ventilator y-piece 502. The ventilator y-piece 502 is coupled to inspiration tubing 506 via a first coupling 510 and expiration tubing 508 via a second coupling 512.

An intubation tube 514 is coupled to the adapter 300. The intubation tube 514 is positioned over the intubation tube mount 404 such that an inner face (not shown) of the intubation tube 514 makes face sharing contact with the intubation tube mount 404, thereby fluidly coupling the adapter 300 to the intubation tube 514.

A breathing circuit 518 may include the intubation tube 514, the inspiration tubing 506, and the expiration tubing 508. The intubation tube 514 may be inserted into the lungs of a patient (e.g., patient 12 in FIG. 1). The inspiration tubing 506 and expiration tubing 508 flow medical gas from the ventilator (e.g., ventilator 20 in FIG. 1-2) through the adapter 300 to the patient via the intubation tube 514. The sensor 430 may sample the gas flow through the breathing circuit 518 and transmit sensor readings to the patient monitor or ventilator control system (e.g., control system 50) for controlling one or more settings of the ventilator.

Generally, mechanical dead space may be defined as the passages of the breathing circuit that extend from the end of the intubation tube or endotracheal tube to the ventilator y-piece. The ventilator pushes medical gas into lungs and out from the lungs. It is a challenge in ventilation systems try to reduce rebreathing expirated gas. For neonatal patients with small lungs, mechanical dead space is relatively big. The patient inhales a substantial proportion of his or her own expirated gases, which causes issues for the patient. Adult patients with reduced lung capacity may be subject to similar challenges. In the example, mechanical dead space in the assembly 500 comprises a combined volume over a first length of passages 520, which includes passages within the adapter 300, and a second length of passages 522, which includes the intubation tube 514. The combined volume of mechanical dead space depends on the size of the intubation tube. The adapter 300 reduces substantial mechanical dead space by integrating the gas sensor adapter and the suction adapter into a single component. In addition to increasing control of medical gas delivered to the patient, reducing mechanical dead space in the adapter increases the accuracy of the ETCO₂ measurement by fully flushing respired gases before supplying fresh gas during ventilator therapy.

FIG. 6 shows an assembly 600 including the adapter 300. The adapter 300 may be the same or similar to the adapter 40 described with reference to FIG. 1, which may be included in an ventilation system (e.g., the ventilation system 10 shown in FIGS. 1-2). Components of the adapter 300 introduced with reference to FIGS. 3-5 are numbered the same and will not be reintroduced. Further, some components of the adapter 300 may not be shown, although it may be understood that they may also be included in adapter 300. For example, the adapter 300 includes the sensor 430 mounted to the integrated region 304 of the junction 302, the ventilator y-piece 502 coupled to the first port 306, and the intubation tube 514 coupled to the third port 310.

A suction catheter system 602 is coupled the adapter 300. In one example, the suction catheter system 602 may be a closed suction catheter system that remains coupled to the ventilation system during operation. The suction catheter system 602 includes a retractable catheter 604 enclosed within a sheath 608, a distal fitting 606, and a proximal fitting 618 (e.g., proximal relative to a suction catheter pump). The suction catheter system 602 may include a pump (e.g., pump 82 in FIG. 1) that, in communication with the retractable catheter 604 and a controller (e.g., suction catheter controller 84 in FIG. 1), may be operated for management of bronchial secretion.

The distal fitting 606 is coupled to the sheath 608. Similarly, the proximal fitting 618 is coupled to the sheath 608 at an opposing end. The distal fitting 606 includes a first coupling 610. A male projection 614 having a radial flange 616 is positioned over the first coupling 610. The suction catheter system 602 is coupled to the adapter 300 via the distal fitting 606 and to the suction catheter pump via the proximal fitting 618. Particularly, the male projection 614 is inserted into catheter receiving collar 408 such that an inner face (not shown) of the catheter receiving collar 408 makes face sharing contact with the male projection 614, thereby fluidly coupling the adapter 300 to the suction catheter system 602. When suction is desired, the retractable catheter 604 may be controlled to extend through the intubation tube 514 into the patient and the pump may be operated to remove secretions via the retractable catheter 604. When suction is not desired, the retractable catheter 604 may be controlled to retract into the male projection 614 and the pump may be shut off

FIG. 7 shows a second example of a gas sensor adapter with suction port, herein an adapter 700, for a ventilation system. The adapter 700 may be the same or similar to the adapter 300 described with reference to FIGS. 3-6, and the adapter 40 described with reference to FIG. 1, which may be included in an ventilation system (e.g., the ventilation system 10 shown in FIGS. 1-2). An axis system 701 is given in FIG. 7 and the figures following indicating an x-axis, a y-axis, and a z-axis. The x-axis may be referred to as a lateral axis, the z-axis may be referred to as a vertical axis, and the y-axis may be referred to as a longitudinal axis.

The adapter 700 comprises a junction 702 with an integrated region 704 for directly releasably receiving a gas concentration sensor (e.g., sensor 430). The adapter 700 comprises a first port 706 fluidly coupled to the junction 702 configured to interface with a ventilator piece, a second port 708 configured to interface with suction catheter system, and a third port 710 configured to interface with an intubation tube (e.g., the ventilator y-piece 502, the suction catheter system 602, and the intubation tube 514 in FIG. 1). The integrated region 704 comprises a sensor mount 760 including a sensor window 762.

The adapter 700 may comprise a manifold 712, wherein the junction 702, the second port 708, and the third port 710 branch off from the manifold 712 to form separate channels. In one example, the junction 702, the second port 708, and the third port 710 branch off from the manifold 712 to form a y-shape. For example, the junction 702 branches off from the manifold 712 at a junction branch 746. The second port 708 branches off from the manifold 712 at a second port branch 748. The third port 710 branches off from the manifold 712 at a third port branch 750. In one example, the junction 702 and the second port 708 branch off from the manifold 712 substantially in parallel. The manifold 712 includes a junction length 730 interposed between the integrated region 704 and the third port 710.

The adapter 700 includes a heat guard 714. The heat guard 714 may reduce contact between the patient (e.g.., patient skin) and a sensor coupled to the adapter 700, which is advantageous as sensors may produce substantial waste heat. For example, the gas sensor may be hotter than 37°C (e.g., normal skin temperature). The waste heat may be particularly challenging for neonatal patients, who have thin and sensitive skin. In general, the heat guard 714 may be a plate made of insulating material, such as plastic, that is positioned between a patient skin and the gas sensor.

In one example, the heat guard 714 is coupled to the junction 702. The heat guard 714 comprises a flexible extension 716 and a guard frame 718 configured to conform to the sensor. For example, the heat guard 714 may frame a gas concentration sensor, such as an IR sensor for measuring FiCO2, EtCO2, real-time CO2 and respiration rate, multi-gas sensor, flow sensor, or other sensor coupled to the adapter 700. In one example, the heat guard 714 may comprise a thermal-blocking coating.

In one example, the flexible extension 716 is joined to an exterior surface 732 of the junction length 730, and on an opposing end, joined to the guard frame 718. The guard frame 718 is formed from a wall 724 that is half circular in shape. The wall 724 comprises a patient facing surface 720 and an opposing, sensor facing surface 722. The guard frame 718 has a first dimension 726 that is approximately twice a second dimension 728, and a third dimension 729 that is approximately half the second dimension 728. In one example, the first dimension 726 and the second dimension 728 may conform to or frame a portion of a sensor, such as an end portion (e.g., see FIG. 8). The third dimension 729 may be similar to a depth of the sensor.

FIG. 8 shows a use example 800 including the adapter 700. The use example 800 illustrates the adapter 700 in a ventilation system (e.g., ventilation system 10 in FIGS. 1-2) assembled with various components, such as the components described with reference to the assemblies in FIGS. 4-6. The assembly components introduced with reference to FIGS. 4-6 are numbered the same and will not be reintroduced. Further, some components of the prior introduces components of the ventilation system may not be shown, although it may be understood that they may also be included in the use example 800. For example, the use example 800 includes the sensor 430 mounted to the integrated region 704 of the junction 702 and the intubation tube 514 sealingly coupled to the third port 710.

The use example 800 shows the adapter 700 fluidly coupled to the patient 12 via the intubation tube 514. The heat guard 714 is configured to frame the sensor 430. For example, the half-circular shape of the wall 724 of the guard frame 718 mimics the half circular shape of a sensor surface 806. However, other configurations are possible. In other examples, the wall of the guard frame 718 may be differently shaped to conform to differently shaped sensors.

With the sensor 430 mounted to the integrated region 704, the guard frame 718 is positioned between the patient 12 and the sensor 430. In one example, the guard frame 718 introduces a distance 808 between a patient skin 804 and the sensor surface 806. For example, the sensor surface 806 may rest on or be spaced apart from the sensor facing surface 722. The patient facing surface 720 may rest on or be spaced apart from the patient skin 804. The distance 808 may increase airflow between the sensor 430 and the patient skin 804, increasing cooling of the sensor 430, and reducing heat transfer from the sensor 430 to the patient skin 804.

FIG. 9 shows a detail view 900 of the adapter 700 as indicated by dashed lines 9-9 in FIG. 7. Components of the adapter 700 introduced with reference to FIGS. 7-8 are numbered the same and will not be reintroduced. For example, the detail view 900 shows the integrated region 704 including the sensor mount 760 and the first port 706. Further, some components of the adapter 700 may not be shown, although it may be understood that they may also be included in adapter 700.

The adapter 700 includes a y-piece mount surface 920, a first sloped segment 922, and a junction sleeve 924. The first sloped segment 922 and the junction sleeve 924 are arranged on opposing ends of the sensor mount 760. The adapter 700 includes a first clip 904 and a second clip 906. The first clip 904 is arranged between the first sloped segment 922 and the sensor mount 760. The second clip 906 is arranged between the sensor mount 760 and the junction sleeve 924.

The first clip 904 and the second clip 906 may be flexible appendages including a first extension 912 and a second extension 914, respectively, joined to the sensor mount 760. A first protrusion 908 and a second protrusion 910 protrude from the first extension 912 and the second extension 914, respectively. The first extension 912 and the second extension 914 are continuous with a wall 916 that frames the sensor mount 760 on three sides. For example, the wall 916 is raised relative to a sensor mount surface 928 by a step 918. When coupled to the adapter 700, the gas concentration sensor is seated on the sensor mount 760 and positioned over the sensor window 762. The first clip 904 and the second clip 906 releasably secure the gas concentration sensor to the sensor mount 760, for example by sealingly clamping a detector and an emitter around opposing sides of the sensor mount 760 (e.g., FIG. 4). The wall 916 is a placement guard that frames the gas sensor on three sides and contributes to correct positioning of the gas sensor on the adapter.

In one example, the sensor window 762 comprises a plastic film 926. When assembled, the gas concentration sensor may direct a beam of IR radiation to the sensor window 762 that passes through the plastic film 926. The IR radiation passes through the plastic film 926 from the emitter on one side to the detector on the other side. If CO₂ is present in the gas sample, the radiation is absorbed, and the detector sends the signal to the control system, calculating the CO₂ concentration by analyzing how much the signal has been reduced.

FIG. 10 shows a view 1000 of the adapter 700. The view 1000 shows interior chambers of the adapter 700 looking down the centerline axis 734 at the first port 706 from 10-10 in FIG. 7. The centerline axis 734 is shown as a dot in the view 1000. In one example, interior chambers of the adapter 300 may be similar to the interior chambers of the adapter 700 shown in the view 1000.

In one example, such as for neonate ventilation, an inner pipe comprising an inner pipe wall 1004, a first inner pipe surface 1002, and a second inner pipe surface 1003 may positioned within the sensor mount 760. Ventilation passes through an inner channel 1001 defined by the first inner pipe surface 1002. The inner pipe further includes a first window 1014 and a second window 1016. The inner pipe reduces dead space in the breathing circuit (e.g., breathing circuit 14 in FIG. 1).

An outer pipe comprising an outer pipe wall 1006, a second outer pipe surface 1008, and the y-piece mount surface 920 of the adaptor extend radially from the centerline axis 734. A counter connector or a y-piece sleeve (e.g., y-piece sleeve 504 in FIG. 5) interfaces with the y-piece mount surface 920, which may be a sealing surface when coupled to the counter connector. The space 1005 between the second outer pipe surface 1008 and the first inner pipe surface 1002 is non-occupied. The space 1005 is a depression, whereas the inner channel 1001 is a channel through the adapter 700. The space 1005 between the inner pipe and the outer pipe is not mechanical dead space because there is practically no ventilation. The inner channel 1001 has an inner pipe radius 1010. In one example, the inner pipe radius 1010 may be 2-4 mm. The first port measured at the 10-10 has a pipe radius 1012, which may be 3-4 times as wide as the inner pipe radius 1010. In some examples, the outer pipe is omitted from the junction length, e.g., junction length 730, such that the second inner pipe surface forms the surface of the junction length. For example, the second inner pipe surface 1003 may be the same or similar to the junction sleeve 924.

In some examples, the disclosed examples of an adapter for a ventilator system, such as, the adapter 40, the adapter 300, and the adapter 700, may be formed from one or more medical grade plastics. For example, the adapter may be formed from one or more of polycarbonate, acrylic, polypropylene, and polyethylene, polyvinyl chloride, and others. The plastic film covering the sensor windows may be very thin (0.1 mm thick, approximately 4 mm area diameter) compared to other parts of the adapter which may be 2 mm thick. In some examples, the adapter may be a one-piece, monolithic member formed, for example, by an injection molding process. As another example, the adapter may be formed by an additive manufacturing process (e.g., 3D printed). In some examples, the sensor windows may be separate films that are attached to the adapter. In some examples, the adapter may be formed from two or more separately molded parts that are connected together at manufacture by a special connection. The special connection could be, for example, a swivel connection. In one example, the swivel connection may be operated to rotate the gas sensor mount (e.g., the integrated region 304, the first port 306) of the adapter in reference to the third port (e.g., third port 310) so that the user may rotate the sensor mount or other part of the adapter to a preferred orientation.

The disclosed adapter may be compatible with a variety of ventilator systems to provide integrated connections for gas measurement, suction, and intubation. As one example, a method for an adapter, such as, the adapter 40, the adapter 300, and the adapter 700, comprises operating a ventilator to flow gas to a patient, the ventilator fluidly coupled to the patient via a first adapter, the adapter comprising a junction with an integrated region for directly releasably receiving a gas concentration sensor, a first port fluidly coupled to the junction configured to interface with a ventilator piece, a second port configured to interface with a suction catheter system, and a third port configured to interface with an intubation tube. In one example, the method may further comprise flowing gas to the patient via the intubation tube, the intubation tube coupled to the third port of the adapter. The method may further comprise operating the gas concentration sensor, the gas concentration sensor fluidly coupled to the integrated region of the adapter and in electronic communication with a control system of the ventilator. As one example, operating the gas concentration sensor may comprise measuring FiCO₂, end tidal CO2, real-time CO₂, and respiration rate. The method may further comprise operating the suction catheter system, the suction catheter system fluidly coupled to the second port of the adapter. In one example, the ventilator may be the ventilator 20, the control system may be the control system 50, the patient may be the patient 12, the intubation tube may be the intubation tube 46, the gas concentration sensor may be the gas concentration sensor 42, and the suction catheter system may be the suction catheter system 80, described with reference to FIGS. 1-2.

The adapter is lightweight, small, and disposable. The adapter is designed to be easy to mount and easy to replace. In one example, a method for an adapter comprises operating a ventilator to flow gas to a patient, the ventilator fluidly coupled to the patient via a first adapter. In response to a signal indicating a degradation status of the first adapter, the adapter may be replaced. Replacing the adapter comprises decoupling the first adapter from the ventilator and the patient and discarding the adapter. The method includes fluidly coupling a second adapter (e.g., a new, replacement adapter) to the ventilator and the patient, and operating the ventilator to flow gas to the patient. In one example, the ventilator may be the ventilator 20, the patient may be the patient 12, and the first adapter may be one of the adapter 40 described with reference to FIGS. 1-2, the adapter 300 described with reference to FIGS. 3-6, and the adapter 700 described with reference to FIGS. 7-10. In one example, the signal may be a gas sensor signal indicating a degraded sensor window, e.g., contaminated. The method may further include releasing the gas concentration sensor from the sensor mount and decoupling the suction catheter from the first adapter, and mounting the aforementioned components to the second adapter.

The adapter 300 for a ventilation system integrates connections for a gas concentration sensor, a suction catheter system, and an intubation tube with reduced mechanical dead space. The integration of gas measurement and suction catheter adapters results in lighter and smaller packaging. As a result, the adapter allows avoiding more invasive methods of CO₂ measurement for neonatal patients. By integrating the concentration sensor directly into the adapter, dead space is reduced and patient lungs are more effectively ventilated. The technical effect of the disclosed systems and methods for an adapter is increased accuracy of gas concentration readings, reduced contamination from the suction catheter, increased control of the delivery of medical gas to the patient, and reduced accumulation of CO₂ inside the patient lungs.

The disclosure also provides support for an adapter for a ventilation system, comprising: a junction with an integrated region for directly releasably receiving a gas concentration sensor, a first port fluidly coupled to the junction configured to interface with a ventilator piece, a second port configured to interface with a suction catheter system, and a third port configured to interface with an intubation tube. In a first example of the system, the system further comprises: a manifold, wherein the junction, the second port, and the third port branch off from the manifold to form separate channels. In a second example of the system, optionally including the first example, a cross sectional area of the manifold decreases from the integrated region of the junction to the third port. In a third example of the system, optionally including one or both of the first and second examples, the junction and the second port branch off from the manifold substantially in parallel. In a fourth example of the system, optionally including one or more or each of the first through third examples the manifold further comprising a junction length interposed between the integrated region and the third port, wherein a first axial length of the junction length is less than a second axial length of the third port. In a fifth example of the system, optionally including one or more or each of the first through fourth examples, the system further comprises: a heat guard coupled to the junction, the heat guard comprising a flexible extension and a guard frame configured to conform to the gas concentration sensor. In a sixth example of the system, optionally including one or more or each of the first through fifth examples the integrated region further comprising a sensor mount surface interposed between a first clip and a second clip, the first clip and the second clip releasably mounting the gas concentration sensor to the sensor mount surface, and a sensor window positioned in the sensor mount surface. In a seventh example of the system, optionally including one or more or each of the first through sixth examples, the manifold is a one-piece, monolithic member. In an eighth example of the system, optionally including one or more or each of the first through seventh examples, the manifold is formed from one or more medical grade plastics.

The disclosure also provides support for a system comprising: a source of medical gas, a ventilator coupled to the source of medical gas, a gas concentration sensor coupled to the ventilator, a suction catheter system coupled to the ventilator, an intubation tube, and an adapter coupled to the ventilator, the gas concentration sensor, and the suction catheter system, and the intubation tube, the adapter comprising a junction with an integrated region for directly releasably receiving the gas concentration sensor, a first port fluidly coupled to the junction configured to interface with the ventilator, a second port configured to interface with the suction catheter system, and a third port configured to interface with the intubation tube. In a first example of the system the adapter further comprising a manifold wherein the junction, the second port, and the third port branch off from the manifold to form separate channels, wherein a cross sectional area of the manifold decreases from the integrated region of the junction to the third port, and wherein the junction and the second port branch off from the manifold substantially in parallel. In a second example of the system, optionally including the first example the manifold further comprising a junction length interposed between the integrated region and the third port, wherein a first axial length of the junction length is less than a second axial length of the third port. In a third example of the system, optionally including one or both of the first and second examples the adapter further comprising a heat guard coupled to the junction, the heat guard comprising a flexible extension and a guard frame configured to conform to the gas concentration sensor. In a fourth example of the system, optionally including one or more or each of the first through third examples the adapter further comprising a sensor mount surface interposed between a first clip and a second clip, the first clip and the second clip releasably mounting the gas concentration sensor to the sensor mount surface, and a sensor window positioned in the sensor mount surface. In a fifth example of the system, optionally including one or more or each of the first through fourth examples, the gas concentration sensor is an infrared sensor comprising an emitter and a detector, the emitter and the detector sealingly clamping around opposing sides of the integrated region. In a sixth example of the system, optionally including one or more or each of the first through fifth examples, the manifold is a one-piece, monolithic member formed from one or more medical grade plastics.

According to an aspect, the entire adapter may be formed as a monolithic structure by one of an injection molding process and a compression molding process. The injection molding process and the compression molding process may include only a single molding step to create the monolithic, one-piece structure. For example, when injection molding, a mold having a cavity, which defines the entirety of the adapter, may be filled with molten plastic to create the complete adapter in a single molding step. That is, the junction including the integrated region, the first port, the second port, the third port, and the manifold may be formed as a rigid, one-piece structure that is unable to be disassembled without fracturing the material of the adapter.

The disclosure also provides support for a method for an adapter, the method comprising: operating a ventilator to flow gas to a patient, the ventilator fluidly coupled to the patient via the adapter, the adapter comprising a junction with an integrated region for directly releasably receiving a gas concentration sensor, a first port fluidly coupled to the junction configured to interface with a ventilator piece, a second port configured to interface with a suction catheter system, and a third port configured to interface with an intubation tube. In a first example of the method, the method further comprises: operating the gas concentration sensor, the gas concentration sensor fluidly coupled to the integrated region of the adapter and in electronic communication with a control system of the ventilator. In a second example of the method, optionally including the first example, operating the gas concentration sensor comprises measuring patient fractional concentration of inspired CO2, end tidal CO2, real-time CO2 concentration, and respiration rate. In a third example of the method, optionally including one or both of the first and second examples, the method further comprises: operating the suction catheter system, the suction catheter system fluidly coupled to the second port of the adapter.

FIGS. 1-10 show example configurations with relative positioning of the various components. If shown directly contacting each other, or directly coupled, then such elements may be referred to as directly contacting or directly coupled, respectively, at least in one example. Similarly, elements shown contiguous or adjacent to one another may be contiguous or adjacent to each other, respectively, at least in one example. As an example, components laying in face-sharing contact with each other may be referred to as in face-sharing contact. As another example, elements positioned apart from each other with only a space there-between and no other components may be referred to as such, in at least one example. As yet another example, elements shown above/below one another, at opposite sides to one another, or to the left/right of one another may be referred to as such, relative to one another. Further, as shown in the figures, a topmost element or point of element may be referred to as a "top" of the component and a bottommost element or point of the element may be referred to as a "bottom" of the component, in at least one example. As used herein, top/bottom, upper/lower, above/below, may be relative to a vertical axis of the figures and used to describe positioning of elements of the figures relative to one another. As such, elements shown above other elements are positioned vertically above the other elements, in one example. As yet another example, shapes of the elements depicted within the figures may be referred to as having those shapes (e.g., such as being circular, straight, planar, curved, rounded, chamfered, angled, or the like). Further, elements shown intersecting one another may be referred to as intersecting elements or intersecting one another, in at least one example. Further still, an element shown within another element or shown outside of another element may be referred as such, in one example.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

As used herein, the term "approximately" is construed to mean plus or minus five percent of the range unless otherwise specified and the term "substantially parallel" means that the elements are sufficiently parallel to be considered parallel to one of ordinary skilled in the art without being perfectly parallel.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. An adapter for a ventilation system, comprising:
a junction (302) with an integrated region (304) for directly releasably receiving a gas concentration sensor, a first port (306) fluidly coupled to the junction configured to interface with a ventilator piece, a second port (308) configured to interface with a suction catheter system, and a third port (310) configured to interface with an intubation tube.

2. The adapter of claim 1, further comprising a manifold (312), wherein the junction, the second port, and the third port branch off from the manifold to form separate channels.

3. The adapter of claim 2, wherein a cross sectional area of the manifold (312) decreases from the integrated region (304) of the junction to the third port (310).

4. The adapter of claim 2, wherein the junction (304) and the second port (308) branch off from the manifold substantially in parallel.

5. The adapter of claim 2, the manifold (312) further comprising a junction length (370) interposed between the integrated region and the third port, wherein a first axial length (323) of the junction length is less than a second axial length (334) of the third port.

6. The adapter of claim 1, further comprising a heat guard (714) coupled to the junction, the heat guard comprising a flexible extension (716) and a guard frame (718) configured to conform to the gas concentration sensor.

7. The adapter of claim 1, the integrated region (304) further comprising a sensor mount surface (360) interposed between a first clip (352) and a second clip (354), the first clip and the second clip releasably mounting the gas concentration sensor to the sensor mount surface, and a sensor window (362) positioned in the sensor mount surface.

8. The adapter of claim 2, wherein the manifold (312) is a one-piece, monolithic member.

9. The adapter of claim 2, wherein the adapter (300) is formed as a one-piece, monolithic structure by one of an injection molding process and a compression molding process.

10. A system comprising:
a source of medical gas (70);
a ventilator (20) coupled to the source of medical gas;
a gas concentration sensor (42) coupled to the ventilator;
a suction catheter system (80) coupled to the ventilator;
an intubation tube (46); and
an adapter (300) coupled to the ventilator, the gas concentration sensor, and the suction catheter system, and the intubation tube, the adapter comprising a junction (302) with an integrated region (304) for directly releasably receiving the gas concentration sensor, a first port (306) fluidly coupled to the junction configured to interface with the ventilator, a second port (308) configured to interface with the suction catheter system, and a third port (310) configured to interface with the intubation tube.

11. The system of claim 10, the adapter (300) further comprising a manifold (312) wherein the junction, the second port, and the third port branch off from the manifold to form separate channels, wherein a cross sectional area of the manifold decreases from the integrated region of the junction to the third port, and wherein the junction and the second port branch off from the manifold substantially in parallel.

12. The system of claim 11, the manifold (312) further comprising a junction length (370) interposed between the integrated region and the third port, wherein a first axial length (323) of the junction length is less than a second axial length (334) of the third port.

13. The system of claim 10, the adapter (300) further comprising a heat guard (714) coupled to the junction, the heat guard comprising a flexible extension (716) and a guard frame (718) configured to conform to the gas concentration sensor.

14. The system of claim 10, the adapter (300) further comprising a sensor mount surface (360) interposed between a first clip (352) and a second clip (354), the first clip and the second clip releasably mounting the gas concentration sensor to the sensor mount surface, and a sensor window positioned in the sensor mount surface.

15. The system of claim 10, wherein the gas concentration sensor (42) is an infrared sensor comprising an emitter and a detector, the emitter and the detector sealingly clamping around opposing sides of the integrated region.
